# EUROPEAN PATENT APPLICATION

(11) **EP 2 913 406 A1**
(43) Date of publication of application: **02.09.2015**
(21) Application number: 15156781.5
(22) Date of filing: 26.02.2015
(51) Int. Cl.: C12Q 1/68

(54) **Nucleic acid amplification method**

(30) Priority: 28.02.2014 JP 2014038659
(71) Applicant: Seiko Epson Corporation, Tokyo 163 (JP)
(72) Inventor: Uehara, Masayuki, Nagano, 392-8502 (JP)
(74) Representative: Hoffmann Eitle

(57) **Abstract**

A nucleic acid amplification method includes: forming a first liquid droplet containing a first reaction reagent for amplifying a first nucleic acid in one vessel containing a silicone oil to which a fluoro-modified silicone resin is added; forming a second liquid droplet containing a second reaction reagent for amplifying a second nucleic acid in the vessel; and performing a nucleic acid amplification reaction in the vessel by fitting the vessel in a nucleic acid amplification reaction apparatus while independently maintaining the first liquid droplet and the second liquid droplet.

## Description

### BACKGROUND

### 1. Technical Field

The present invention relates to a nucleic acid amplification method.

### 2. Related Art

In recent years, as a result of development of technologies utilizing genes, medical treatments utilizing genes such as genetic diagnosis or genetic therapy are drawing attention, and in addition, many methods utilizing genes in determination of breed varieties or breed improvement have also been developed in agricultural and livestock industries. As technologies for utilizing genes, nucleic acid amplification methods such as a PCR (Polymerase Chain Reaction) method are widely used. Nowadays, the PCR method has become an indispensable technology in elucidation of information on biological materials.

The PCR method is a method of amplifying a target nucleic acid by subjecting a reaction mixture containing a target nucleic acid to be amplified and a reagent to a thermal cycle. The thermal cycle is a process of subjecting a reaction mixture to two or more stages of temperatures periodically. In the PCR method, a method of performing a two- or three-stage thermal cycle is generally used.

In recent years, a method of simultaneously amplifying two types of target DNAs such as influenza virus DNAs has been effectively developed (see Ozdemir M. , Yavru S. , Baysal B. "Comparison of the detection of influenza A and B viruses by different methods." J. Int. Med. Res. 2012; 40(6): 2401-8). In this case, two types of target DNAs are amplified in one tube, and therefore, a reaction is generally performed by adding two types of target DNAs and two types of PCR reagents such as primers to one solution placed in the tube.

### SUMMARY

An advantage of some aspects of the invention is to provide a novel nucleic acid amplification method capable of efficiently performing multiple types of nucleic acid amplification reactions in one vessel.

An aspect of the invention is directed to a method of performing multiple nucleic acid amplification reactions in one vessel, the vessel containing a mixture of a silicone oil and a fluoro-modified silicone resin, the method including: forming a first liquid droplet containing a first reaction reagent for amplifying a first nucleic acid in the vessel; forming a second liquid droplet containing a second reaction reagent for amplifying a second nucleic acid in the vessel; and performing a nucleic acid amplification reaction in the vessel by fitting the vessel in a nucleic acid amplification reaction apparatus while independently maintaining the first liquid droplet and the second liquid droplet. The first liquid droplet and the second liquid droplet may contain the same type of nucleic acid sample or may contain different types of nucleic acid samples. The fluoro-modified silicone resin may be XS66-C1191. Further, the first liquid droplet may contain a first fluorescent dye, and the second liquid droplet may contain a second fluorescent dye which is different from the first fluorescent dye. Further, the addition amount of the fluoro-modified silicone resin may be 1% by mass or more and 50% by mass or less.

Another aspect of the invention is directed to a nucleic acid amplification reaction vessel, which is for performing multiple nucleic acid amplification reactions, and contains: a silicone oil to which a fluoro-modified silicone resin is added; a first liquid droplet containing a reaction reagent for amplifying a first nucleic acid; and a second liquid droplet containing a reaction reagent for amplifying a second nucleic acid. The fluoro-modified silicone resin may be XS66-C1191. Further, the first liquid droplet may contain a first fluorescent dye, and the second liquid droplet may contain a second fluorescent dye which is different from the first fluorescent dye. Further, the addition amount of the fluoro-modified silicone resin may be 1% by mass or more and 50% by mass or less.

Still another aspect of the invention is directed to a nucleic acid amplification apparatus fitted with any of the vessels described above.

According to the aspects of the invention, a novel nucleic acid amplification method capable of efficiently performing multiple types of nucleic acid amplification reactions in one vessel.

### BRIEF DESCRIPTION OF THE DRAWINGS

The invention will be described with reference to the accompanying drawings, wherein like numbers reference like elements.
FIGS. 1A and 1B are perspective views of an elevating-type PCR apparatus according to one embodiment of the invention. FIG. 1A shows a state in which a lid is closed, and FIG. 1B shows a state in which the lid is opened.
FIG. 2 is an exploded perspective view of a main body of the elevating-type PCR apparatus according to one embodiment of the invention.
FIG. 3 is a cross-sectional view of a tube according to one embodiment of the invention.
FIGS. 4A and 4B are cross-sectional views schematically showing the cross section taken along the line A-A in FIG. 1A of the main body of the elevating-type PCR apparatus according to one embodiment of the invention. FIG. 4A shows a first arrangement, and FIG. 4B shows a second arrangement.
FIG. 5 is a view showing the results of an examination of the relationship between the addition amount of XS66-C1191 and the appearance of the formation of a liquid droplet in one example of the invention.
FIG. 6 is a view showing the results of multiplex PCR performed with two liquid droplets in one example of the invention.
FIG. 7 is a view showing the results of multiplex PCR performed with one liquid droplet in one example of the invention.

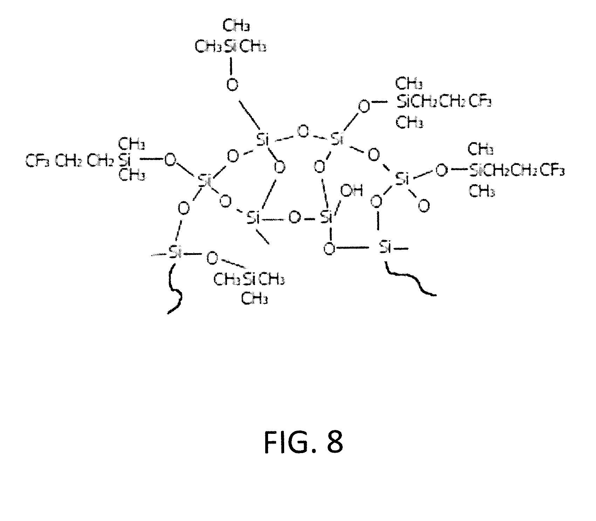

Fig. 8 shows the structure of the fluoro-modified silicone resin XS66-C1191.

### DESCRIPTION OF EXEMPLARY EMBODIMENTS

The object, characteristics, and advantages of the invention as well as the idea thereof will be apparent to those skilled in the art from the description given herein, and the invention can be easily reproduced by those skilled in the art based on the description given herein. It is to be understood that the embodiments, specific examples, etc. of the invention described below are to be taken as preferred embodiments of the invention, and are presented for illustrative or explanatory purposes and are not intended to limit the invention. It is further apparent to those skilled in the art that various changes and modifications may be made based on the description given herein within the intent and scope of the invention disclosed herein.

When there is no particular description in the embodiments or examples, methods described in standard protocols such as J. Sambrook, E. F. Fritsch & T. Maniatis (Ed.), Molecular cloning, a laboratory manual (3rd edition), Cold Spring Harbor Press, Cold Spring Harbor, New York (2001); F. M. Ausubel, R. Brent, R. E. Kingston, D. D. Moore, J. G. Seidman, J. A. Smith, K. Struhl (Ed.), Current Protocols in Molecular Biology, John Wiley & Sons Ltd. , or modified or altered methods thereof are used. When using a commercially available reagent kit or a measuring apparatus, unless otherwise particularly indicated, a protocol attached thereto is used.

### (1) Method of Performing Nucleic Acid Amplification Reaction

The method according to the invention includes: forming a first liquid droplet containing a first reaction reagent for amplifying a first nucleic acid in one vessel containing a silicone oil to which a fluoro-modified silicone resin is added; forming a second liquid droplet containing a second reaction reagent for amplifying a second nucleic acid in the vessel; and performing a nucleic acid amplification reaction in the vessel by fitting the vessel in a nucleic acid amplification reaction apparatus while independently maintaining the first liquid droplet and the second liquid droplet. According to this method, multiple nucleic acid amplification reactions can be performed in one vessel. In this method, the nucleic acid amplification reaction may be performed using a reaction reagent for amplifying a nucleic acid, and it is not always necessary to amplify the nucleic acid after the reaction.

The concentration of the fluoro-modified silicone resin is not particularly limited, but is preferably 1% by mass or more and 50% by mass or less, more preferably 1% by mass or more and 20% by mass or less, further more preferably 1% by mass or more and 5% by mass or less. The type of the fluoro-modified silicone resin is not particularly limited, but is preferably XS66-C1191 (Momentive, Inc.). The type of the silicone oil is not particularly limited, and examples thereof include dimethyl silicone oil, methyl hydrogen silicone oil, methylphenyl silicone oil, and cyclic dimethyl silicone oil. The nucleic acid amplification method is not particularly limited and may be any method as long as the method is commonly used by those skilled in the art, but it is preferably an elevating-type PCR apparatus (see, for example, JP-A-2012-115208). By incorporating a fluorescent dye having a different emission wavelength in each liquid droplet, even when emitted fluorescence is measured simultaneously, each nucleic acid can be distinguished, and thus, so-called multiplex PCR or the like can be achieved.

Here, the first nucleic acid and the second nucleic acid which can be amplified with the respective reaction reagents may be the same or different. In the case where the first nucleic acid and the second nucleic acid are the same type of nucleic acid and the first reaction reagent and the second reaction reagent are the same reaction reagent, for example, by incorporating a sample containing the nucleic acid in both liquid droplets and performing an amplification reaction, it is possible to obtain a reaction product in a larger amount than in the case where a reaction is performed with one liquid droplet. In the case where the first nucleic acid and the second nucleic acid are the same type of nucleic acid and the first reaction reagent and the second reaction reagent are different from each other, for example, by incorporating a sample containing the nucleic acid in both liquid droplets and performing an amplification reaction, it is possible to examine which reaction reagent can more efficiently achieve amplification or the like. According to this, for example, by preparing primers having a partially different sequence for the same gene, it is possible to examine which primer functions more efficiently.

In the case where the first nucleic acid and the second nucleic acid are different from each other and the first reaction reagent and the second reaction reagent are the same reaction reagent, that is, in the case where the reaction reagent can amplify multiple nucleic acids, for example, it is possible to simultaneously amplify two types of nucleic acids in one vessel and moreover with one type of reaction reagent.

In the case where the first nucleic acid and the second nucleic acid are different from each other and preferred conditions for the first reaction reagent and the second reaction reagent are different with respect to each of the nucleic acids, for example, in the case where a sample in which it is unknown whether a nucleic acid contained therein is the first nucleic acid or the second nucleic acid is incorporated in both liquid droplets, it is possible to examine whether the nucleic acid is the first nucleic acid or the second nucleic acid. According to this, for example, it is possible to use them for identifying the type of influenza virus or the like. Alternatively, by incorporating a sample containing the first nucleic acid and a sample containing the second nucleic acid in liquid droplets separately, it is possible to simultaneously amplify two types of nucleic acids in one vessel.

In order to examine whether background amplification occurs due to a primer dimer or a nucleic acid or the like mixed in the reaction reagent in one liquid droplet or both liquid droplets, a reaction may be performed without incorporating a nucleic acid sample.

A reagent contained in the reaction reagent is not particularly limited, and examples thereof include reagents commonly used in nucleic acid amplification such as a buffer, a DNA polymerase, dNTPs, a primer, and depending on the amplification reaction, a probe. A primer or a probe may be fluorescently labeled.

The vessel used here is preferably a tube, and may be a micro-centrifuge tube or a tube designed for PCR.

### (2) Nucleic Acid Amplification Reaction Vessel

According to one embodiment of the invention, a nucleic acid amplification reaction vessel contains: a silicone oil to which a fluoro-modified silicone resin is added; a first liquid droplet containing a reaction reagent for amplifying a first nucleic acid; and a second liquid droplet containing a reaction reagent for amplifying a second nucleic acid. By using this vessel, as described in (1), various nucleic acid amplification reactions can be performed for one or multiple nucleic acid samples. In the same manner as in (1), this vessel is preferably a tube, and may be a micro-centrifuge tube or a tube designed for PCR.

### (3) Example of Structure of Elevating-Type PCR Apparatus

Hereinafter, one example of an elevating-type PCR apparatus will be described in detail.

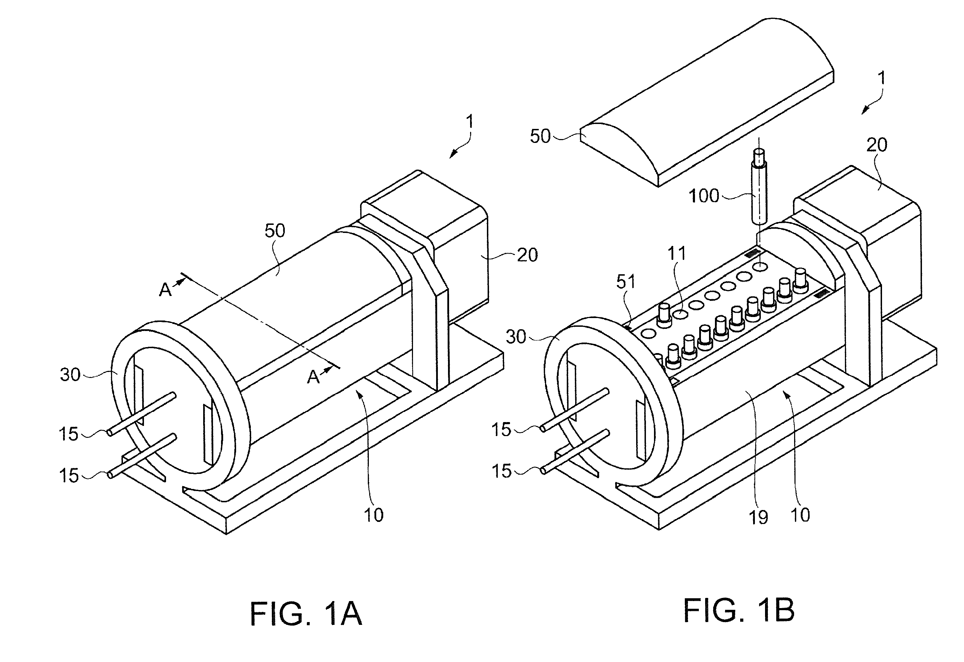

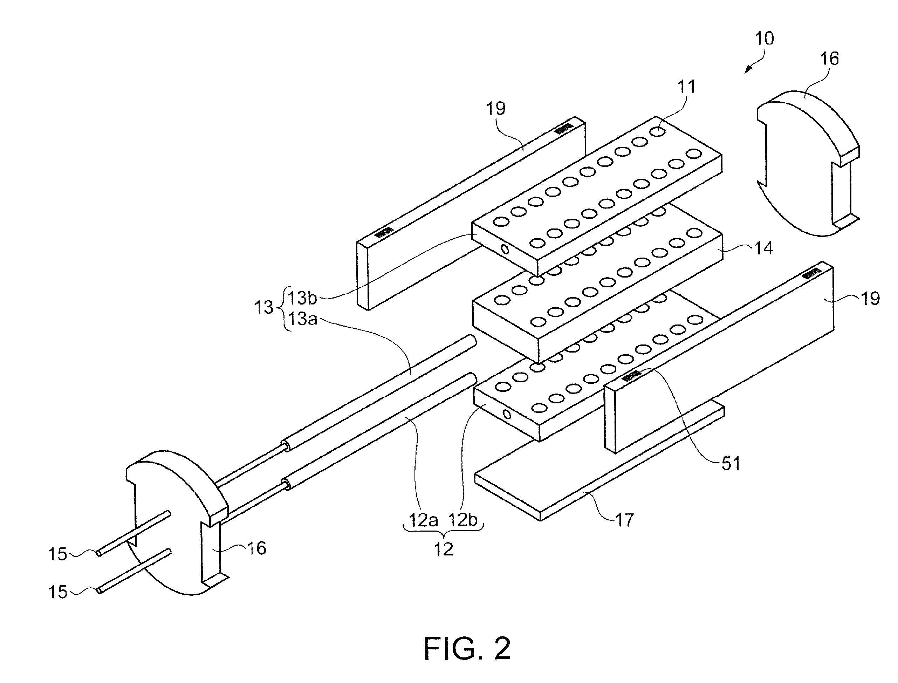

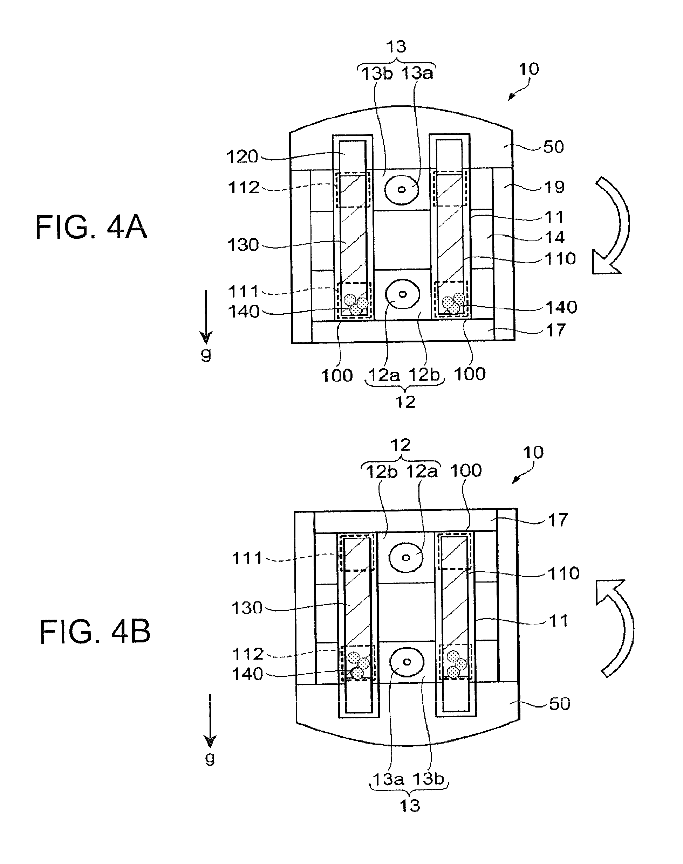

FIGS. 1A and 1B show one example of an elevating-type PCR apparatus 1. FIG. 1A shows a state in which a lid 50 of the elevating-type PCR apparatus 1 is closed, and FIG. 1B shows a state in which the lid 50 of the elevating-type PCR apparatus 1 is opened and a nucleic acid amplification reaction tube 100 is fitted in a fitting section 11. FIG. 2 is an exploded perspective view of a main body 10 of the elevating-type PCR apparatus 1 according to the embodiment. FIG. 4A is a cross-sectional view schematically showing the cross section taken along the line A-A in FIG. 1A of the main body 10 of the elevating-type PCR apparatus 1 according to the embodiment.

This elevating-type PCR apparatus 1 includes the main body 10 and a driving mechanism 20 as shown in FIG. 1A. As shown in FIG. 2, the main body 10 includes the fitting section 11, a first heating section 12, and a second heating section 13. A spacer 14 is provided between the first heating section 12 and the second heating section 13. In the main body 10 of this embodiment, the first heating section 12 is disposed on the side of a bottom plate 17, and the second heating section 13 is disposed on the side of the lid 50. In the main body 10 of this embodiment, the first heating section 12, the second heating section 13, and the spacer 14 are fixed by a flange 16, the bottom plate 17, and a fixing plate 19.

The fitting section 11 is configured to fit the nucleic acid amplification reaction tube 100, which will be described later. As shown in FIGS. 1B and 2, the fitting section 11 of this embodiment has a slot structure in which the nucleic acid amplification reaction tube 100 is inserted and fitted, and is configured such that the nucleic acid amplification reaction tube 100 is inserted into a hole penetrating a first heat block 12b of the first heating section 12, the spacer 14, and a second heat block 13b of the second heating section 13. The number of the fitting sections 11 may be more than one, and in the example shown in FIG. 1B, twenty fitting sections 11 are provided for the main body 10.

This elevating-type PCR apparatus 1 includes a structure in which the nucleic acid amplification reaction tube 100 is held at a predetermined position with respect to the first heating section 12 and the second heating section 13. More specifically, as shown in FIGS. 4A and 4B, in a flow channel 110 constituting the nucleic acid amplification reaction tube 100, which will be described later, a first region 111 can be heated by the first heating section 12 and a second region 112 can be heated by the second heating section 13. In this embodiment, a structure that defines the position of the nucleic acid amplification reaction tube 100 is the bottom plate 17, and as shown in FIG. 4A, by inserting the nucleic acid amplification reaction tube 100 to a position in contact with the bottom plate 17, the nucleic acid amplification reaction tube 100 can be held at a predetermined position with respect to the first heating section 12 and the second heating section 13.

When the nucleic acid amplification reaction tube 100 is fitted in the fitting section 11, the first heating section 12 heats the first region 111 of the nucleic acid amplification reaction tube 100, which will be described later, to a first temperature. In the example shown in FIG. 4A, in the main body 10, the first heating section 12 is disposed at a position where the first region 111 of the nucleic acid amplification reaction tube 100 is heated.

The first heating section 12 may include a mechanism that generates heat and a member that transfers the generated heat to the nucleic acid amplification reaction tube 100. In the example shown in FIG. 2, the first heating section 12 includes the first heater 12a and the first heat block 12b. In this embodiment, the first heater 12a is a cartridge heater, and is connected to an external power source (not shown) through a conductive wire 15. The first heater 12a is inserted into the first heat block 12b, and the first heat block 12b is heated by heat generated by the first heater 12a. The first heat block 12b is a member that transfers heat generated by the first heater 12a to the nucleic acid amplification reaction tube 100. In this embodiment, the first heat block 12b is an aluminum block.

When the nucleic acid amplification reaction tube 100 is fitted in the fitting section 11, the second heating section 13 heats the second region 112 of the nucleic acid amplification reaction tube 100 to a second temperature different from the first temperature. In the example shown in FIG. 4A, in the main body 10, the second heating section 13 is disposed at a position where the second region 112 of the nucleic acid amplification reaction tube 100 is heated. As shown in FIG. 2, the second heating section 13 includes a second heater 13a and the second heat block 13b. The second heating section 13 is configured in the same manner as the first heating section 12 except that the region of the nucleic acid amplification reaction tube 100 to be heated and the heating temperature are different from those for the first heating section 12.

In this embodiment, the temperatures of the first heating section 12 and the second heating section 13 are controlled by a temperature sensor (not shown) and a control section (not shown), which will be described later. The temperatures of the first heating section 12 and the second heating section 13 are preferably set so as to heat the nucleic acid amplification reaction tube 100 to a desired temperature. In this embodiment, by controlling the first heating section 12 at the first temperature and the second heating section 13 at the second temperature, the first region 111 of the nucleic acid amplification reaction tube 100 can be heated to the first temperature, and the second region 112 can be heated to the second temperature. The temperature sensor in this embodiment is a thermocouple.

The driving mechanism 20 is a mechanism that drives the fitting section 11, the first heating section 12, and the second heating section 13. In this embodiment, the driving mechanism 20 includes a motor (not shown) and a drive shaft (not shown), and the drive shaft is connected to the flange 16 of the main body 10. The drive shaft in this embodiment is provided perpendicular to the longitudinal direction of the fitting section 11, and when the motor is activated, the main body 10 is rotated about the drive shaft as the axis of rotation.

The elevating-type PCR apparatus 1 of this embodiment includes the control section (not shown). The control section controls at least one of the first temperature, the second temperature, the first period, the second period, and the number of thermal cycles, which will be described later. In the case where the control section controls the first period or the second period, the control section controls the operation of the driving mechanism 20, thereby controlling the period in which the fitting section 11, the first heating section 12, and the second heating section 13 are held in a predetermined arrangement. The control section may have mechanisms different in each item to be controlled, or may control all items collectively. However, the control section in the elevating-type PCR apparatus 1 of this embodiment is an electronic control system and controls all of the above-described items. The control section of this embodiment includes a processor such as CPU (not shown) and a storage device such as an ROM (Read Only Memory) or an RAM (Random Access Memory). In the storage device, various programs, data, etc. for controlling the above-described respective items are stored. Further, the storage device has a work area for temporarily storing data in processing, processing results, etc. of various processes.

As shown in the example of FIGS. 2 and 4A, in the main body 10 of this embodiment, the spacer 14 is provided between the first heating section 12 and the second heating section 13. The spacer 14 of this embodiment is a member that holds the first heating section 12 or the second heating section 13. In this embodiment, the spacer 14 is a heat insulating material, and in the example shown in FIG. 4A, the fitting section 11 penetrates the spacer 14.

The main body 10 of this embodiment includes the fixing plate 19. The fixing plate 19 is a member that holds the fitting section 11, the first heating section 12, and the second heating section 13. In the example shown in FIGS. 1B and 2, two fixing plates 19 are fitted in the flanges 16, and the first heating section 12, the second heating section 13, and the bottom plate 17 are fixed by the fixing plates 19.

The elevating-type PCR apparatus 1 of this embodiment includes the lid 50. In the example shown in FIGS. 1A and 4A, the fitting section 11 is covered with the lid 50. The lid 50 may be fixed to the main body 10 by a fixing section 51. In this embodiment, the fixing section 51 is a magnet. As shown in the example of FIGS. 1B and 2, a magnet is provided on a surface of the main body 10 which comes into contact with the lid 50. Although not shown in FIGS. 1B and 2, a magnet is provided also for the lid 50 in a place with which the magnet of the main body 10 comes into contact. When the fitting section 11 is covered with the lid 50, the lid 50 is fixed to the main body 10 with a magnetic force.

It is preferred that the fixing plate 19, the bottom plate 17, the lid 50, and the flange 16 are formed using a heat insulating material.

### (3) Example of Thermal Cycling Process Using Elevating-type PCR Apparatus

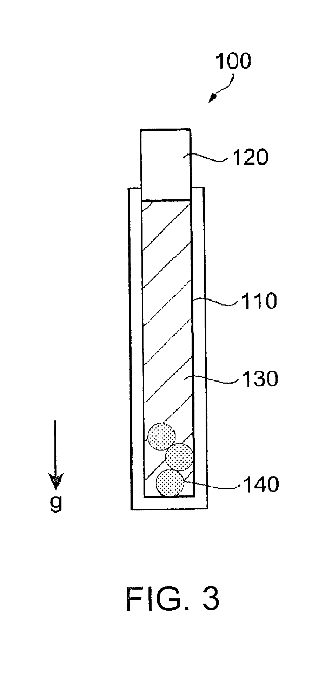

FIG. 3 is a cross-sectional view of the nucleic acid amplification reaction tube 100 according to the embodiment. FIGS. 4A and 4B are cross-sectional views schematically showing the cross section taken along the line A-A in FIG. 1A of the elevating-type PCR apparatus 1 according to the embodiment. FIGS. 4A and 4B show a state in which the nucleic acid amplification reaction tube 100 is fitted in the elevating-type PCR apparatus 1. FIG. 4A shows a first arrangement, and FIG. 4B shows a second arrangement. Hereinafter, first, the nucleic acid amplification reaction tube 100 according to the embodiment will be described, and thereafter, the thermal cycling process using the elevating-type PCR apparatus 1 according to the embodiment in the case of using the nucleic acid amplification reaction tube 100 will be described.

As shown in the example of FIG. 3, the nucleic acid amplification reaction tube 100 according to the embodiment includes a flow channel 110 and a sealing section 120. The flow channel 110 is filled with a reaction mixture 140 and a silicone oil 130 which has a specific gravity smaller than the reaction mixture 140 and is immiscible with the reaction mixture 140, and sealed with the sealing section 120.

The flow channel 110 is formed such that the reaction mixture 140 moves in close proximity to opposed inner walls. Here, the term "opposed inner walls" of the flow channel 110 refers to two regions of a wall surface of the flow channel 110 having an opposed positional relationship. The phrase "in close proximity to" refers to a state in which the distance between the reaction mixture 140 and the wall surface of the flow channel 110 is close, and includes a case where the reaction mixture 140 is in contact with the wall surface of the flow channel 110. Therefore, the phrase "the reaction mixture 140 moves in close proximity to opposed inner walls" refers to that "the reaction mixture 140 moves in a state of being close in distance to both of the two regions of a wall surface of the flow channel 110 having an opposed positional relationship", that is, the reaction mixture 140 moves along the opposed inner walls.

In the example shown in Fig. 3, the outer shape of the nucleic acid amplification reaction tube 100 is a cylindrical shape, and the flow channel 110 is formed in the direction along a center axis (the vertical direction in Fig. 3) therein. The shape of the flow channel 110 is a tubular shape having a circular cross section in the direction perpendicular to the longitudinal direction of the flow channel 110, that is, in the direction perpendicular to the direction of the movement of the reaction mixture 140 in a region in the flow channel 110 (this cross section is defined as the "cross section" of the flow channel 110). Therefore, in the nucleic acid amplification reaction tube 100 of this embodiment, the opposed inner walls of the flow channel 110 are regions including two points on the wall surface of the flow channel 110 constituting the diameter of the cross section of the flow channel 110, and the reaction mixture 140 moves in the longitudinal direction of the flow channel 110 along the opposed inner walls.

The first region 111 of the nucleic acid amplification reaction tube 100 is a partial region of the flow channel 110 which is heated to the first temperature by the first heating section 12. The second region 112 is a partial region of the flow channel 110 which is different from the first region 111 and is heated to the second temperature by the second heating section 13. In the nucleic acid amplification reaction tube 100 of this embodiment, the first region 111 is a region including one end portion in the longitudinal direction of the flow channel 110, and the second region 112 is a region including the other end portion in the longitudinal direction of the flow channel 110. In the example shown in FIGS. 4A and 4B, a region surrounded by the dotted line including an end portion on the proximal side of the sealing section 120 of the flow channel 110 is the second region 112, and a region surrounded by the dotted line including an end portion on the distal side of the sealing section 120 is the first region 111.

The flow channel 110 contains the silicone oil 130 and multiple liquid droplets of the reaction mixture 140. Since the silicone oil 130 is immiscible with the reaction mixture, that is, has a property that it is not mixed with the reaction mixture, the reaction mixture 140 is held in a state of a liquid droplet in the silicone oil 130 as shown in Fig. 3. The reaction mixture 140 is located in a lowermost portion of the flow channel 110 with respect to the gravitational direction because it has a specific gravity larger than the silicone oil 130. The reaction mixture 140 is a liquid containing a reaction reagent. When the reaction is PCR, the reaction mixture 140 contains a DNA (a target nucleic acid) to be amplified by PCR, a DNA polymerase, a primer, a probe, a salt, and the like.

Hereinafter, with reference to FIGS. 4A and 4B, the thermal cycling process using the elevating-type PCR apparatus 1 according to the embodiment will be described. In FIGS. 4A and 4B, the direction indicated by the arrow g (in the downward direction in the drawing) is the gravitational direction. In this embodiment, a case where shuttle PCR (two-stage temperature PCR) is performed will be described as an example of the thermal cycling process. The respective steps described below are shown as an example of the thermal cycling process, and according to need, the order of the steps may be changed, two or more steps may be performed continuously or concurrently, or a step may be added.

The shuttle PCR is a method of amplifying a nucleic acid in a reaction mixture by subjecting the reaction mixture to a two-stage temperature process at a high temperature and a low temperature repeatedly. In the process at a high temperature, denaturation of a double-stranded DNA occurs and in the process at a low temperature, annealing (a reaction in which a primer is bound to a single-stranded DNA) and elongation (a reaction in which a complementary strand to the DNA is formed by using the primer as a starting point) occur.

In general, in the shuttle PCR, the high temperature is a temperature between 80°C and 100°C and the low temperature is a temperature between 50°C and 70°C. The processes at the respective temperatures are performed for a predetermined period, and a period of maintaining the reaction mixture at a high temperature is generally shorter than a period of maintaining the reaction mixture at a low temperature. For example, the period for the process at a high temperature may be about 1 to 10 seconds, and the period for the process at a low temperature may be about 10 to 60 seconds, or a period longer than this range may be adopted depending on the condition of the reaction.

Since the appropriate period, temperature, number of cycles (number of times of repetition of the process at a high temperature and the process at a low temperature) varies depending on the type or amount of a reagent to be used, it is preferred to determine an appropriate protocol in consideration of the type of a reagent or the amount of the reaction mixture 140 before performing the reaction.

First, the nucleic acid amplification reaction tube 100 is fitted in the fitting section 11. In this embodiment, the nucleic acid amplification reaction tube 100, in which the reaction mixture 140 is introduced into the flow channel 110 previously filled with the silicone oil 130, and thereafter the flow channel 110 is sealed with the sealing section 120, is fitted in the fitting section 11. The introduction of the reaction mixture 140 can be performed using a micropipette, an ink-jet dispenser, or the like. In a state in which the nucleic acid amplification reaction tube 100 is fitted in the fitting section 11, the first heating section 12 is in contact with the nucleic acid amplification reaction tube 100 at a place including the first region 111 and the second heating section 13 is in contact with the nucleic acid amplification reaction tube 100 at a place including the second region 112.

Here, the arrangement of the fitting section 11, the first heating section 12, and the second heating section 13 is the first arrangement. As shown in FIG. 4A, in the first arrangement, the first region 111 of the nucleic acid amplification reaction tube 100 is located in a lowermost portion of the flow channel 110 with respect to the gravitational direction. In the first arrangement, the first region 111 is located in a lowermost portion of the flow channel 110 with respect to the gravitational direction, and therefore, the reaction mixture 140 having a specific gravity larger than the silicone oil 130 is located in the first region 111. In this embodiment, after the nucleic acid amplification reaction tube 100 is fitted in the fitting section 11, the fitting section 11 is covered with the lid 50, and then the elevating-type PCR apparatus 1 is activated.

Subsequently, the nucleic acid amplification reaction tube 100 is heated by the first heating section 12 and the second heating section 13. The first heating section 12 and the second heating section 13 heat different regions of the nucleic acid amplification reaction tube 100 to different temperatures. That is, the first heating section 12 heats the first region 111 to the first temperature, and the second heating section 13 heats the second region 112 to the second temperature. Accordingly, a temperature gradient in which the temperature gradually changes between the first temperature and the second temperature is formed between the first region 111 and the second region 112 of the flow channel 110. Here, a temperature gradient in which the temperature is decreased from the first region 111 to the second region 112 is formed. The thermal cycling process of this embodiment is the shuttle PCR, and therefore, the first temperature is set to a temperature suitable for the denaturation of a double-stranded DNA, and the second temperature is set to a temperature suitable for the annealing and elongation.

Since the arrangement of the fitting section 11, the first heating section 12, and the second heating section 13 is the first arrangement, when the nucleic acid amplification reaction tube 100 is heated, the reaction mixture 140 is heated to the first temperature. When the first period has elapsed, the main body 10 is driven by the driving mechanism 20, and the arrangement of the fitting section 11, the first heating section 12, and the second heating section 13 is switched over from the first arrangement to the second arrangement. The second arrangement is an arrangement in which the second region 112 is located in a lowermost portion of the flow channel 110 with respect to the gravitational direction. In other words, the second region 112 is a region located in a lowermost portion of the flow channel 110 with respect to the gravitational direction when the fitting section 11, the first heating section 12, and the second heating section 13 are in a predetermined arrangement different from the first arrangement. In the elevating-type PCR apparatus 1 of this embodiment, by the control of the control section, the driving mechanism 20 rotatively drives the main body 10. When the flanges 16 are rotatively driven by the motor by using the drive shaft as the axis of rotation, the fitting section 11, the first heating section 12, and the second heating section 13 which are fixed to the flanges 16 are rotated. Since the drive shaft is a shaft extending in the direction perpendicular to the longitudinal direction of the fitting section 11, when the drive shaft is rotated by the activation of the motor, the fitting section 11, the first heating section 12, and the second heating section 13 are rotated. In the example shown in FIGS. 4A and 4B, the main body 10 is rotated at 180°. By doing this, the arrangement of the fitting section 11, the first heating section 12, and the second heating section 13 is switched over from the first arrangement to the second arrangement.

Here, the positional relationship between the first region 111 and the second region 112 with respect to the gravitational direction is opposite from that of the first arrangement, and therefore, the reaction mixture 140 moves from the first region 111 to the second region 112 by the gravitational force. After the arrangement of the fitting section 11, the first heating section 12, and the second heating section 13 has reached the second arrangement, when the operation of the driving mechanism 20 is stopped, the fitting section 11, the first heating section 12, and the second heating section 13 are held in the second arrangement. When the second period has elapsed in the second arrangement, the main body 10 is rotated again. A nucleic acid amplification reaction is performed by rotating the main body 10 while switching over between the first arrangement and the second arrangement in this manner until the number of thermal cycles has reached a predetermined number of cycles.

### (4) Another Embodiment

According to this embodiment of the invention, it is possible to independently form water-soluble liquid droplets in one tube containing a silicone oil to which XS66-C1191 is added in an amount of 1% by mass or more and 50% by mass or less. As described in Examples, even after the tube is subjected to a harsh reaction such as a nucleic acid amplification reaction, the independence of the liquid droplets is maintained. Therefore, it is considered that also in a reaction other than the nucleic acid amplification reaction, by forming multiple liquid droplets in a silicon oil, and subjecting different samples to the reaction in each liquid droplet, a multiplex reaction can be efficiently performed. Here, the conditions for XS66-C1191 and the silicone oil are as described above.

### Examples

### (1) Addition Amount of XS66-C1191 and Formation of Liquid Droplets

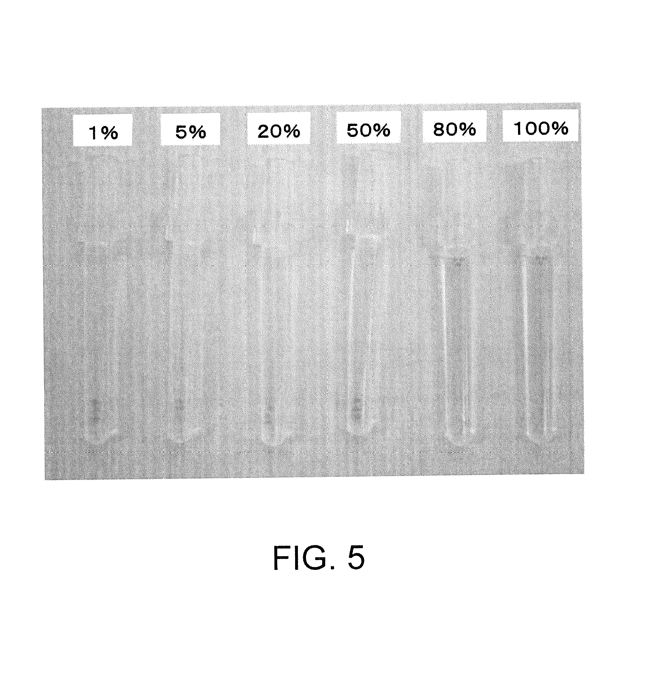

The nucleic acid amplification reaction tube of this Example has a cylindrical outer shape, and has a flow channel in the shape of a cylinder with an inner diameter of 2 mm and a length of 25 mm. First, the nucleic acid amplification reaction tube was filled with 130 µm of a solution obtained by adding a fluoro-modified silicone resin (XS66-C1191, manufactured by Momentive, Inc.) in an amount of 1, 5, 20, 50, 80, or 100% by mass to dimethyl silicone oil (KF-96L-2cs, manufactured by Shin-Etsu Silicone Co., Ltd.). Then, by using a micropipette, three droplets (1 µL each) of water colored with a color marker (loading buffer, manufactured by Takara Bio, Inc.) were introduced into the flow channel. As a result, as shown in FIG. 5, in the solutions obtained by adding a fluoro-modified silicone resin in an amount of 1, 5, 20, or 50% by mass to dimethyl silicone oil, each reaction mixture was maintained in a state of a spherical liquid droplet with a diameter of about 1.5 mm, and the liquid droplets did not mix with each other and existed independently.

### (2) PCR with Two Liquid Droplets

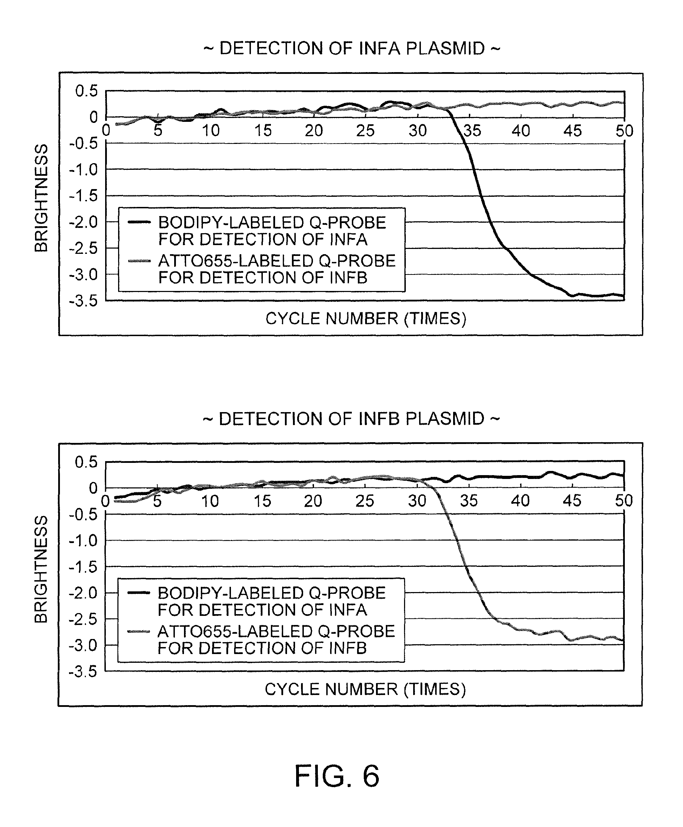

Subsequently, PCR was performed by using the same tube and introducing reaction mixtures having the following composition into KF-96L-2cs containing 20% by mass of XS66-C1191 in an amount of 1 µL each. Incidentally, an amplification reaction was performed with respect to the case where InfA plasmid was added as the plasmid to both liquid droplets (upper graph in FIG. 6: Detection of InfA plasmid) and the case where InfB plasmid was added as the plasmid to both liquid droplets (lower graph in FIG. 6: Detection of InfB plasmid), and the reaction was specifically caused only in a reaction mixture 1 or a reaction mixture 2.

Reaction Mixture 1:

| | |
|---|---|
| 50x Titanium Taq | 0.4 |
| 10x buffer | 1.0 |
| dNTP (10 mM) | 0.2 |
| InfA forward primer (5 µM) | 1.0 |
| InfA reverse primer (5 µM) | 0.3 |
| InfA Qprobe (2 µM) | 0.25 |
| plasmid | 1.0 |
| DW | up to 10.0 (unit: µL) |

### Reaction Mixture 2:

| | |
|---|---|
| 50x Titanium Taq | 0.4 |
| 10x buffer | 1.0 |
| dNTP (10 mM) | 0.2 |
| InfB forward primer (5 µM) | 0.3 |
| InfB reverse primer (5 µM) | 1.0 |
| InfB Qprobe (2 µM) | 0.25 |
| plasmid | 1.0 |
| DW | up to 10.0 (unit: µL) |

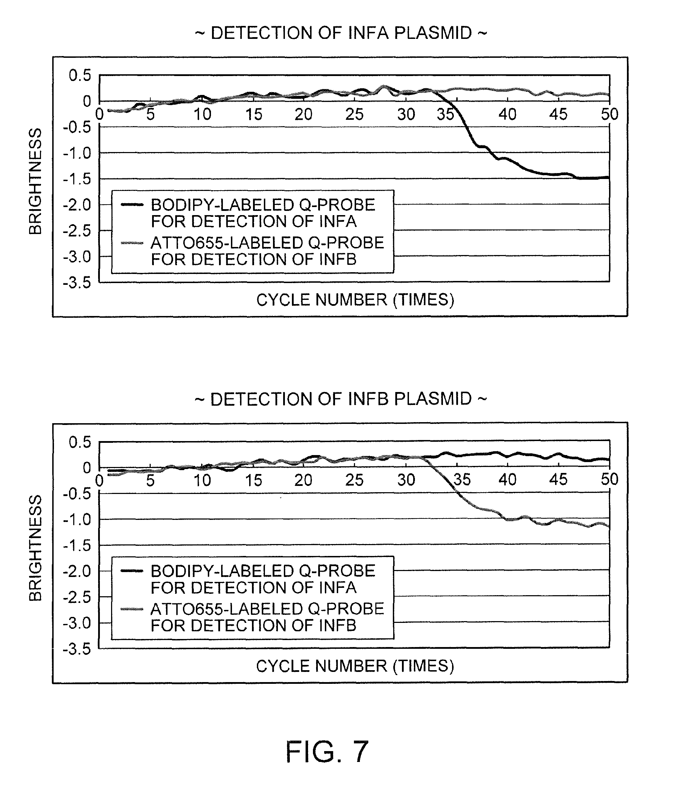

As a control experiment, PCR was performed with one liquid droplet. A reaction mixture used has the following composition (reaction mixture 3). Incidentally, an amplification reaction was performed with respect to the case where InfA plasmid was added as the plasmid (upper graph in FIG. 7: Detection of InfA plasmid) and the case where InfB plasmid was added as the plasmid (lower graph in FIG. 7: Detection of InfB plasmid).

### Reaction Mixture 3:

| | |
|---|---|
| 50x Titanium Taq | 0.8 |
| 10x buffer | 1.0 |
| dNTP (10 mM) | 0.2 |
| InfA forward primer (5 µM) | 1.0 |
| InfA reverse primer (5 µM) | 0.3 |
| InfA Qprobe (2 µM) | 0.25 |
| InfB forward primer (5 µM) | 0.3 |
| InfB reverse primer (5 µM) | 1.0 |
| InfB Qprobe (2 µM) | 0.25 |
| Plasmid | 1.0 |
| DW | up to 10.0 (unit: µL) |

The sequences of the primer and the probe are as follows.
InfA forward primer: 5 ' -GACCRATCCTGTCACCTCTGAC-3' (SEQ ID NO: 1)
InfA reverse primer: 5'-AGGGCATTYTGGACAAAKCGTCTA-3' (SEQ ID NO: 2)
InfA Qprobe: 5'-Bodipy-CACAAATCCTAAAATTCCCT-3' (SEQ ID NO: 3)
InfB forward primer: 5'-TCCTCAACTCACTCTTCGAGCG -3' (SEQ ID NO: 4)
InfB reverse primer: 5'-CGGTGCTCTTGACCAAATTGG-3' (SEQ ID NO: 5)
InfB Qprobe: 5'-Atto655- CATTCAAAGCCAATTCGA-3' (SEQ ID NO: 6)

After the reaction mixture was treated at 98°C for 10 seconds, 50 thermal cycles were performed under the following conditions: 98°C for 5 seconds, and 52°C for 20 seconds.

Even after the 50 thermal cycles were performed, the two liquid droplets did not mix with each other and existed independently. The measurement results of fluorescence during the reaction are shown in FIG. 6 (in the case of two liquid droplets) and FIG. 7 (in the case of one liquid droplet) .

As apparent from the comparison between FIG. 6 and FIG. 7, the amplification curve started to rise about one cycle earlier and the brightness change amount was significantly larger in the case where PCR was performed with two liquid droplets than in the case where PCR was performed with one liquid droplet. In this manner, PCR amplification efficiency is higher in the case where PCR is performed with multiple liquid droplets.

## Claims

1. A method of performing multiple nucleic acid amplification reactions in one vessel, the vessel containing a mixture of a silicone oil and a fluoro-modified silicone resin, the method comprising:
forming a first liquid droplet containing a first reaction reagent for amplifying a first nucleic acid in the mixture in the vessel ;
forming a second liquid droplet containing a second reaction reagent for amplifying a second nucleic acid in the mixture in the vessel; and
performing a nucleic acid amplification reaction by fitting the vessel in a nucleic acid amplification reaction apparatus while independently maintaining the first liquid droplet and the second liquid droplet.

2. The method according to claim 1, wherein the first liquid droplet and the second liquid droplet contain the same type of nucleic acid sample.

3. The method according to claim 1, wherein the first liquid droplet and the second liquid droplet contain different types of nucleic acid samples.

4. The method according to any of claims 1-3, wherein the fluoro-modified silicone resin is XS66-C1191 (Momentive, Inc.).

5. The method according to any of claims 1-4, wherein
the first liquid droplet contains a first fluorescent dye, and
the second liquid droplet contains a second fluorescent dye which is different from the first fluorescent dye.

6. The method according to any of claims 1-5, wherein the addition amount of the fluoro-modified silicone resin is 1% by mass or more and 50% by mass or less.

7. A vessel which is for performing multiple nucleic acid amplification reactions, wherein the vessel contains:
a silicone oil to which a fluoro-modified silicone resin is added;
a first liquid droplet containing a reaction reagent for amplifying a first nucleic acid; and
a second liquid droplet containing a reaction reagent for amplifying a second nucleic acid.

8. The vessel according to claim 7, wherein the fluoro-modified silicone resin is XS66-C1191 (Momentive, Inc.).

9. The vessel according to claim 7 or claim 8, wherein
the first liquid droplet contains a first fluorescent dye, and
the second liquid droplet contains a second fluorescent dye which is different from the first fluorescent dye.

10. The vessel according to any of claims 7-9, wherein the addition amount of the fluoro-modified silicone resin is 1% by mass or more and 50% by mass or less.

11. A nucleic acid amplification apparatus, wherein the vessel according to any of claims 7-10 is fitted therein.


SEQUENCE LISTING

<110> Seiko Epson Corporation

<120> Nucleic acid amplification method

<130> J017565301

<160> 6

<170> PatentIn version 3.5

<210> 1
<211> 22
<212> DNA
<213> Artificial Sequence

<220>
<223> primer

<400> 1
gaccratcct gtcacctctg ac                                                22

<210> 2
<211> 24
<212> DNA
<213> Artificial Sequence

<220>

<223> primer

<400> 2

agggcattyt ggacaaakcg tcta 24

<210> 3

<211> 20

<212> DNA

<213> Artificial Sequence

<220>

<223> probe

<400> 3

cacaaatcct aaaattccct 20

<210> 4

<211> 22

<212> DNA

<213> Artificial Sequence

<220>

<223> primer

<400> 4

tcctcaactc actcttcgag cg                                                22

<210> 5

<211> 21

<212> DNA

<213> Artificial Sequence

<220>

<223> primer

<400> 5

cggtgctctt gaccaaattg g                                                 21

<210> 6

<211> 18

<212> DNA

<213> Artificial Sequence

<220>

<223> probe

<400> 6

cattcaaagc caattcga                                                     18

SEQUENCE LISTING

<110> Seiko Epson Corporation

<120> Nucleic acid amplification method

<130> J017565301, HE178946

<150> JP2014-038659
<151> 2014-02-28

<160> 6

<170> BiSSAP 1.3

<210> 1
<211> 22
<212> DNA
<213> Artificial Sequence

<220>
<223> primer

<400> 1
gaccratcct gtcacctctg ac                                                22

<210> 2
<211> 24
<212> DNA
<213> Artificial Sequence

<220>
<223> primer

<400> 2
agggcattyt ggacaaakcg tcta                                              24

<210> 3
<211> 20
<212> DNA
<213> Artificial Sequence

<220>
<223> probe

<400> 3
cacaaatcct aaaattccct                                                   20

<210> 4
<211> 22
<212> DNA
<213> Artificial Sequence

<220>
<223> primer

<400> 4
tcctcaactc actcttcgag cg                                                22

<210> 5
<211> 21
<212> DNA
<213> Artificial Sequence

<220>
<223> primer

<400> 5
cggtgctctt gaccaaattg g                                                21


<210> 6
<211> 18
<212> DNA
<213> Artificial Sequence

<220>
<223> probe

<400> 6
cattcaaagc caattcga                                                    18